# EUROPEAN PATENT SPECIFICATION

(11) **EP 4 163 394 B1**
(45) Date of publication and mention of the grant of the patent: **20.11.2024**
(21) Application number: 22199908.9
(22) Date of filing: 05.10.2022
(51) Int. Cl.: C12Q 1/6883

(54) **METHOD FOR DETERMINING TOUGH AND DRY MEAT SYNDROME (TDMS)**
VERFAHREN ZUR BESTIMMUNG DES ZÄH- UND TROCKENFLEISCHSYNDROMS (TDMS)
PROCÉDÉ DE DÉTERMINATION DU SYNDROME DE VIANDE DURE ET SÈCHE (TDM)

(30) Priority: 05.10.2021 EP 21201071
(43) Date of publication of application: 12.04.2023
(73) Proprietor: Université de Liège, 4000 Liège (BE); Universiteit Gent, 9000 Gent (BE)
(72) Inventor: CHARLIER, Carole, 4000 Liège (BE); GEORGES, Michel, 4000 Liège (BE); DRUET, Tom, 4000 Liège (BE); DE SMET, Stefaan, 9000 Gent (BE); ROMBOUTS, Toon, 9000 Gent (BE)
(74) Representative: De Clercq & Partners

(56) References cited:
- DROGEMULLER C ET AL: "Identification of a missense mutation in the bovine ATP2A1 gene in congenital pseudomyotonia of Chianina cattle: An animal model of human Brody disease", GENOMICS, ACADEMIC PRESS, SAN DIEGO, US, vol. 92, no. 6, 1 December 2008 (2008-12-01), pages 474 - 477, XP025671485, ISSN: 0888-7543, [retrieved on 20080925], DOI: 10.1016/J.YGENO.2008.07.014
- LEONARDO MURGIANO ET AL: "Pseudomyotonia in Romagnola cattle caused by novel ATP2A1 mutations", BMC VETERINARY RESEARCH, BIOMED CENTRAL, LONDON, GB, vol. 8, no. 1, 9 October 2012 (2012-10-09), pages 186, XP021137324, ISSN: 1746-6148, DOI: 10.1186/1746-6148-8-186
- MASCARELLO FRANCESCO ET AL: "Structural study of skeletal muscle fibres in healthy and pseudomyotonia affected cattle", ANNALS OF ANATOMY, JENA, DE, vol. 207, 19 May 2016 (2016-05-19), pages 21 - 26, XP029704019, ISSN: 0940-9602, DOI: 10.1016/J.AANAT.2016.05.002
- GEORGES MICHEL ET AL: "Harnessing genomic information for livestock improvement", NATURE REVIEWS GENETICS, NATURE PUBLISHING GROUP, GB, vol. 20, no. 3, 4 December 2018 (2018-12-04), pages 135 - 156, XP036703129, ISSN: 1471-0056, [retrieved on 20181204], DOI: 10.1038/S41576-018-0082-2

## Description

### Field of the invention

This invention relates to methods for the detection of bovine that are affected by "Dry Meat Syndrome" (DMS) or "Tough and Dry Meat Syndrome" (TDMS), an inherited defect with autosomal, partially dominant inheritance. The present invention provides methods for determining whether a bovine is homozygous or heterozygous for mutations that cause DMS or DMTS by analyzing its genomic DNA or its RNA. The methods include obtaining a sample of material containing genomic DNA or RNA from the bovine, and genotyping or sequencing said nucleic acid for the presence of one or more hypomorphic mutations in the bovine ATPase Sarcoplasmic/Endoplasmic Reticulum Ca2+ Transporting 1 (*ATP2A1*) gene such as the *R143W* mutation.

### Description of the background art

### DNA testing in livestock

DNA testing is increasingly used in routine practice in animal breeding and production. DNA testing in livestock comes mainly in two forms: (i) genotyping animals at specific loci and for specific variants that have been shown to have a major effect on a trait of interest whether in homozygotes only (recessive inheritance) or also in heterozygotes (dominant inheritance, which can be partial if the phenotype of heterozygotes is less pronounced than that of homozygotes), and (ii) genotyping animals for thousands of variants scattered across the genome and use this information to compute Genomic Breeding Values (GBV) for traits of interest, i.e. Genomic Selection (GS). The first approach mainly applies to genetic defects which are often determined by one or a small number of loci (monogenic or oligogenic inheritance). The second approach mainly applies to economically important traits (such as milk or meat yield) which are typically determined by a large number of loci (polygenic inheritance). In some populations, the genetic determinism (sometimes referred to as "architecture") of economically important traits may involve one or a small number of loci/variants with major effect on the trait of interest, as well as many polygenes with individually very small effects on the trait of interest. Variations of GS allow to incorporate the information of these variants with large effects to compute GBV. An example of a locus/variant with a major effect on a polygenic economically important trait are the loss-of-function mutations in the myostatin gene that cause "double-muscling" in cattle.

Animals are usually not selected for one trait only but rather for a weighted combination of several economically important traits (f.i. meat yield, fertility, disease resistance, ...), referred to as "Breeding Goal" (BG). The GBV for individual traits can be combined in a GBV for the composite BG. Major gene effects on economically important traits with polygenic architecture can be incorporated when computing GBV for a BG. Additionally, the economic impact of monogenic defects can be estimated and the genetic defect added as a separate trait to the BG with a weight that is based on its economic impact.

In summary, DNA testing in livestock is at present mainly used to (i) identify and eliminate carriers of severe monogenetic defects including embryonic lethals, and (ii) determine the GBV for weighted combinations of polygenic economically important traits and select the animals with the highest "breeding worth". These two goals will increasingly be merged into one process by defining BG that take the economic impact of genetic defects into account (Georges et al. 2018).

In the above: (i) locus/loci refers to a region/regions in the genome that encompass genetic variants that affect a trait of interest, and (ii) genetic variants refers to alternative sequences that coexist in a population at such a locus. Such genetic variants constitute "alleles" in the classical genetic sense. These alternative sequences may affect the function of genes located in their vicinity and thereby affect phenotypes or traits that depend on the function of the corresponding gene(s).

### Selecting for meat quality

In beef cattle selection for economically important traits has thus far primarily focused on yield traits (f.i. carcass yield), feed efficiency, health and fertility traits. Meat quality has been recognized as an important target for decades. It includes color, water holding capacity, tenderness, degree of marbling, and taste. Thus far, it has only seldomly been added to BG for at least two reasons. The first is that meat quality remains difficult and expensive to measure, and the second is that consumers have to be willing to pay premiums for higher quality meat while economic incentives have to propagate throughout the entire production chain. While meat quality traits are likely to essentially conform to a polygenic architecture, there exist at least two examples of genetic variants with major effect on meat quality in pigs. The first is a missense variant (*R615C*) in the ryanodine receptor gene (*RYR*) that codes for a calcium release channel in the sarcoplasmic reticulum (Fujii et al., 1991). Animals that are homozygous for this mutation suffer potentially lethal malignant hyperthermia upon use of volatile anesthetics or exposure to stress-full conditions (including transport to the slaughterhouse). Even if not succumbing to a crisis, the sensory and technological quality of the meat of affected animals is inferior and referred to as P(ale)-S(oft)-E(xudative) meat causing major economic loss. Heterozygous animals may show minor or intermediate forms of the PSE syndrome. The *R615C* RYR mutation originated from the Piétrain breed which is extensively used as sire line. It has been eliminated from most commercial pig population by "marker assisted selection" (MAS). The second is a dominant *R200Q* mutation in the *PRKAG3* gene known as the "RN" mutation that is increasing glycogen content in the meat and negatively affects processing yield (Milan et al., 2000). It originates from the Hampshire breed, and has also been largely eliminated by MAS. Of interest, both mutations increase meat yield (including in heterozygotes) accounting for their - at some point - high frequencies in commercial pig populations.

### Early reports of a novel meat quality defect in Belgian Blue Cattle: the dry meat syndrome (DMS)

In 2015, Belgian meat cutters reported the occasional observation of meat cuts that feel tense and were described as "tough and dry". Most of the affected muscles were located in high-value cuts in the hindquarter: i.a. *m. glutaeobiceps* (silverside), *m. semimembranosus* (topside) and *m. quadriceps femoris* (top rump). In the worst cases i.a. *m. longissimus dorsi* (eye muscle), *m. subscapularis* (underblade) and *m. teres major* (shoulder tender) were also affected. The corresponding carcasses were systematically from heavily muscled (DS class according to SEUROP scheme ≥ 93%; Anonymous, 1981) adult (age ≥ 4 years) Belgian Blue cows. The condition is now referred to as "Dry Meat Syndrome" (DMS) or "Tough and Dry Meat Syndrome (TDMS).

### Summary of the invention

The technical problem underlying the present invention was to provide means and methods that allow for a selective and convenient diagnosis of DMS or TDMS in cattle.

The solution to said technical problem is achieved by the embodiments characterized in the claims. In particular, provided herein is a method for determining whether a bovine is affected by or susceptible to Tough and Dry Meat Syndrome (TDMS), comprising determining the presence or absence of at least one genetic variant or mutation in the *ATP2A1* gene in a nucleic acid from the bovine.

The present inventors found that TDMS can be diagnosed on the basis of the detection of one or more loss-of-function and/or hypomorphic *ATP2A1* mutations, such as the R143W mutation. The present invention provides for the first time the identity of mutations causing DMS or TDMS in cattle.

### Brief description of the figures

The teaching of the application is illustrated by the following Figures which are to be considered as illustrative only and do not in any way limit the scope of the claims.

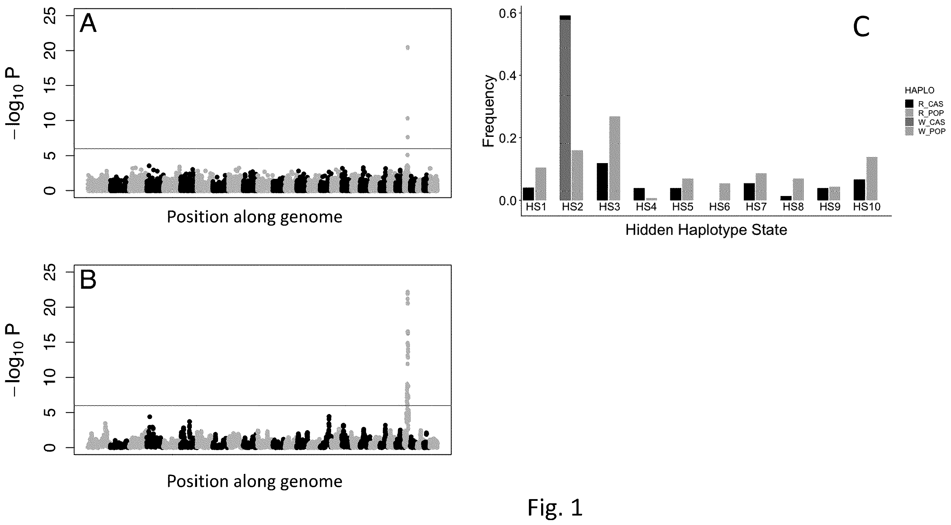

**Figure 1****: Genome-wide association study (GWAS) for DMS using 38 affected animals and 1,058 age-matched controls representative of the general BBC population. (A-B)** Manhattan plots obtained with a model interrogating single SNPs (A) or ten hidden haplotype states (HS) centered on the considered SNP (B). The horizontal red line marks the genome-wide significance threshold. Alternating colors (black and grey) mark the limits between the 29 autosomes. **(C)** Frequency of the ten HS in the 38 cases (black) and the 1,058 controls (gray) at peak position. The red and orange bars mark the proportion of the HS that is carrying the mutant *143W ATP2A1* variant in cases and the general population (=controls), respectively.

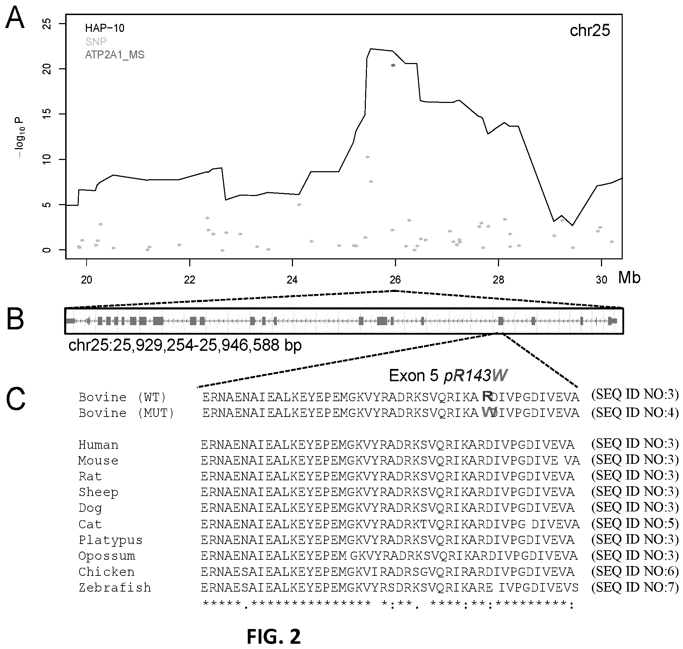

**Figure 2****: Local association signal and evolutionary conservation of the *R143* amino acid (aa) in *ATP2A1* exon 5. (A)** Association signals for a single SNP model (grey dots) and a ten hidden-states model (black line) in a 10 Mb region of chromosome 25 encompassing the *ATP2A1* gene and *pR143W* causative variant (red dot). **(B)** Gene model for the *ATP2A1* gene (adapted from UCSC browser). **(C)** *ATP2A1* exon5 protein alignment across vertebrates highlighting the *pR143W* amino acid change; * marks invariant aa.

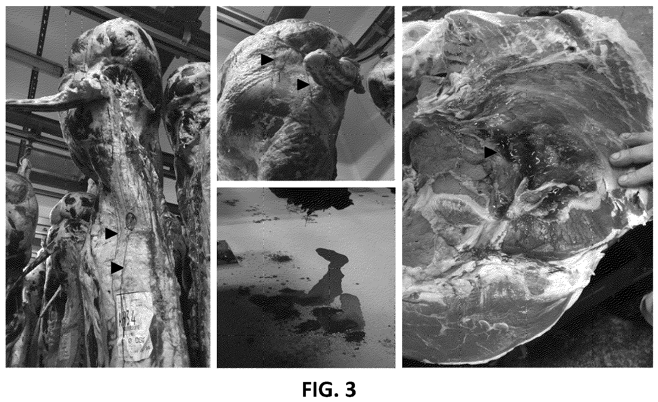

**Figure 3****:** Main macroscopic characteristics of DMS or TDMS carcasses.

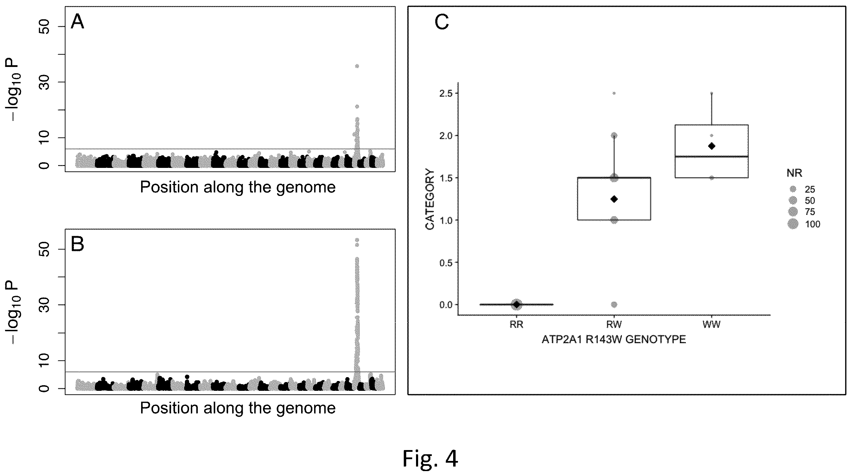

**Figure 4****: (A-B)** Confirmation of the association between the *R143W ATP2A1* variant and DMS or TDMS in an independent cohort comprising 116 cases and 127 negative controls. **(C)** Distribution of DMS or TDMS severity score as a function of R143W genotype.

### Description of the invention

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. Where reference is made to embodiments as comprising certain elements or steps, this encompasses also embodiments which consist essentially of the recited elements or steps.

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

As used herein, the term "and/or" when used in a list of two or more items, means that any one of the listed items can be employed by itself or any combination of two or more of the listed items can be employed. For example, if a list is described as comprising group A, B, and/or C, the list can comprise A alone; B alone; C alone; A and B in combination; A and C in combination, B and C in combination; or A, B, and C in combination.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, aspects, structures or characteristics may be combined in any suitable manner, as would be apparent to one of ordinary skill in the art from this disclosure, in one or more embodiments. Also embodiments described for an aspect of the invention may be used for another aspect of the invention and can be combined.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects.

The present invention relates to a method for determining whether a bovine is affected by or susceptible to Tough and Dry Meat Syndrome (TDMS), comprising determining the presence or absence of at least one genetic variant or mutation in the *ATP2A1* gene in a nucleic acid from the bovine.

A particular embodiment is a method for determining the status of a bovine with regards to DMS by genotyping said bovine for DMS-causing *ATP2A1* variants by analyzing its genomic DNA, the method comprising the steps of:
a. obtaining a sample of material containing said genomic DNA from the bovine,
b. extracting the DNA from said sample,
c. genotyping said DNA for one or more DMS-causing *ATP2A1* variants, and
d. determining whether said animal is homozygous, compound heterozygous or heterozygous for DMS-causing *ATP2A1* variants.

A further particular embodiments is a method for determining the status of a bovine with regards to DMS by genotyping said bovine for the *R143W* DMS-causing *ATP2A1* variant by analyzing its genomic DNA, the method comprising the steps of:
a. obtaining a sample of material containing said genomic DNA from the bovine,
b. extracting the DNA from said sample,
c. genotyping said DNA for the *R143W* DMS-causing *ATP2A1* variant, and
d. determining whether said animal is homozygous, or heterozygous for the *R143W* DMS-causing *ATP2A1* variants.

A further particular embodiments is a method for determining the status of a bovine with regards to DMS by genotyping said bovine for one or more of the *R559C, R164H, G211V, G286V and*/*or G284V A TP2A 1* variants by analyzing its genomic DNA, the method comprising the steps of:
a. obtaining a sample of material containing said genomic DNA from the bovine,
b. extracting the DNA from said sample,
c. genotyping said DNA for the *R559C* and/or the *R164H* and/or the *G211V* and/or the G286V and/or the *G284V ATP2A1* variants, and
d. determining whether said animal is homozygous, compound heterozygous or heterozygous for DMS-causing *ATP2A1* variants.

Another particular embodiment is a method for determining the status of a bovine with regards to DMS by genotyping said bovine for DMS-causing *ATP2A1* variants by analyzing its genomic RNA, the method comprising the steps of:
a. obtaining a sample of material containing said genomic RNA from the bovine,
b. extracting the RNA from said sample,
c. genotyping said RNA for one or more DMS-causing *ATP2A1* variants, and
d. determining whether said animal is homozygous, compound heterozygous or heterozygous for DMS-causing *ATP2A1* variants.

A further particular embodiments is a method for determining the status of a bovine with regards to DMS by genotyping said bovine for the *R143W* DMS-causing *ATP2A1* variant by analyzing its genomic RNA, the method comprising the steps of:
a. obtaining a sample of material containing said genomic RNA from the bovine,
b. extracting the RNA from said sample,
c. genotyping said RNA for the *R143W* DMS-causing *ATP2A1* variant, and
d. determining whether said animal is homozygous, or heterozygous for the *R143W* DMS-causing *ATP2A1* variants.

A further particular embodiments is a method for determining the status of a bovine with regards to DMS by genotyping said bovine for one or more of the *R559C, R164H, G211V, G286V and*/*or G284V A TP2A 1* variants by analyzing its genomic RNA, the method comprising the steps of:
a. obtaining a sample of material containing said genomic RNA from the bovine,
b. extracting the RNA from said sample,
c. genotyping said RNA for the *R559C* and/or the *R164H* and/or the *G211V* and/or the G286V and/or the *G284V A TP2A 1* variants, and
d. determining whether said animal is homozygous, compound heterozygous or heterozygous for DMS-causing *ATP2A1* variants.

In embodiments, the sample is selected from the group consisting of blood, sperm, hair roots, milk, body fluids and/or tissues including nucleated cells.

In embodiments, the bovine is selected from the species bos taurus.

In embodiments, the bovine is selected from the group consisting of Belgian Blue Cattle, Charolais, Roodbont, Piedmontese, Chianina or Romagnola.

A further aspect relates to a method for marker assisted selection or genomic selection for meat quality using the method of the invention.

As used herein, the terms "Dry Meat Syndrome" (DMS) and "Tough and Dry Meat Syndrome" (TDMS) are used as synonyms. The terms refer to a condition in bovine characterized by an increased loss of fluid from the carcass (also referred to as "drip") during the cooling process (i.e. the period between slaughter and cutting) resulting in inferior meat quality. In embodiments, a bovine affected by TDMS may exhibit exudate at the surface of the *m. semimembranosus,* and a dry and tough feeling of the *m. adductor femoris* and inner parts of the *m. glutaeobiceps* and *m. semimembranosus.* In further embodiments, the TDMS affected bovine may exhibit a dry and tough feeling of the *m. quadriceps femoris, m. semitendinosus, m. longissimus dorsi, m. subscapularis and m. teres major.*

As used herein "inferior meat quality" means a significant worsening of one or more measurable meat quality traits below the mean of a given population. Meat quality traits which may be considered include but are not limited to the following:
Color, e.g. as determined using the Commission Internationale de 1'Eclairage (CIE) L*a*b* scores for respectively lightness, redness and yellowness color of the meat.

### Color stability

Texture, e.g. as determined by TPA: higher hardness, gumminess, chewiness, shear force, and shorter sarcomere length are inferior.

Moisture loss: reduced EZ-drip, thawing and cooking loss are not inferior but result from the prior drip losses from the carcass.

These meat quality traits are examples of those generally accepted by those of skill in the art and techniques for measuring these meat quality traits are commonly known. Measurement of a meat quality trait may be e.g. on a sample of the *m. glutaeobiceps.*

As used herein, L*, a*, and b* refer to dimensions of a three-dimensional color space which is standardized to reflect how color is perceived by humans. The L* dimension corresponds to lightness (a value of zero being black, a value of 100 being white), the a* dimension corresponds to relative levels of green and red (a negative value being green, a positive value being red), and the b* dimension corresponds to relative levels of blue and yellow (a negative value being blue, a positive value being yellow).

The acronym "TPA" as used herein stands for Texture Profile Analysis and is composed of one or more rheological parameters as described hereinafter. The term "hardness" as used herein is intended to mean the force required to obtain a deformation of a body. The firmness measurement unit may be expressed in Newtons. The terms firmness and hardness can be used interchangeably. The term "cohesiveness" as used herein is intended to mean the strength of the internal bonds making up the body of the meat. It can be defined as the molecular force between particles within a body or substance that acts to unite them. Cohesiveness is a ratio of two firmness measurements and has therefore no units. The term "springiness" as used herein is intended to mean the rate at which deformed meat goes back to its original undeformed state after removal of the force. The springiness is the property of a substance that enables it to change its length, volume, or shape in direct response to a force affecting such a change and to recover its original form upon the removal of the force. The terms springiness and elasticity can be used interchangeably. The term "adhesiveness" as used herein is intended to mean the force necessary to overcome the attractive forces between the surface of a matter and the surface of another material with which it is in contact. The adhesiveness is the attractive molecular force that tends to hold together unlike bodies when they are in contact. The measurement unit of adhesiveness may be expressed in Newtons. The term " gumminess" is defined as the energy required to disintegrate meat. It is related to the primary parameters of firmness and cohesiveness. It may be expressed in Newtons. The term " chewiness" is defined herein as the energy required to masticate meat. It is related to the primary parameters of firmness, cohesiveness and springiness. It may be expressed in Newtons.

The term "nucleic acid" as used herein typically refers to a polymer (preferably a linear polymer) of any length composed essentially of nucleoside units. A nucleoside unit commonly includes a heterocyclic base and a sugar group. Heterocyclic bases may include *inter alia* purine and pyrimidine bases such as adenine (A), guanine (G), cytosine (C), thymine (T) and uracil (U), which are widespread in naturally-occurring nucleic acids, other naturally-occurring bases (e.g., xanthine, inosine, hypoxanthine), as well as chemically or biochemically modified (e.g., methylated), non-natural or derivatised bases. Exemplary modified nucleobases include, without limitation, 5-substituted pyrimidines, 6-azapyrimidines and N-2, N-6 and O-6 substituted purines, including 2-aminopropyladenine, 5-propynyluracil and 5-propynylcytosine. In particular, 5-methylcytosine substitutions have been shown to increase nucleic acid duplex stability. Sugar groups may include *inter alia* pentose (pentofuranose) groups such as preferably ribose and/or 2-deoxyribose common in naturally-occurring nucleic acids, or arabinose, 2-deoxyarabinose, threose or hexose sugar groups, as well as modified or substituted sugar groups (such as, without limitation, 2'-O-alkylated, e.g., 2'-O-methylated or 2'-O-ethylated sugars such as ribose; 2'-O-alkyloxyalkylated, e.g., 2'-O-methoxyethylated sugars such as ribose; or 2'-O,4'-C-alkylene-linked, e.g., 2'-O,4'-C-methylene-linked or 2'-O,4'-C-ethylene-linked sugars such as ribose; 2'-fluoro-arabinose, etc.). Nucleoside units may be linked to one another by any one of numerous known inter-nucleoside linkages, including *inter alia* phosphodiester linkages common in naturally-occurring nucleic acids, and further modified phosphate- or phosphonate-based linkages such as phosphorothioate, alkyl phosphorothioate such as methyl phosphorothioate, phosphorodithioate, alkylphosphonate such as methylphosphonate, alkylphosphonothioate, phosphotriester such as alkylphosphotriester, phosphoramidate, phosphoropiperazidate, phosphoromorpholidate, bridged phosphoramidate, bridged methylene phosphonate, bridged phosphorothioate; and further siloxane, carbonate, sulfamate, carboalkoxy, acetamidate, carbamate such as 3'-N-carbamate, morpholino, borano, thioether, 3'-thioacetal, and sulfone internucleoside linkages. Preferably, inter-nucleoside linkages may be phosphate-based linkages including modified phosphate-based linkages, such as more preferably phosphodiester, phosphorothioate or phosphorodithioate linkages or combinations thereof. The term "nucleic acid" also encompasses any other nucleobase containing polymers such as nucleic acid mimetics, including, without limitation, peptide nucleic acids (PNA), peptide nucleic acids with phosphate groups (PHONA), locked nucleic acids (LNA), morpholino phosphorodiamidate-backbone nucleic acids (PMO), cyclohexene nucleic acids (CeNA), tricyclo-DNA (tcDNA), and nucleic acids having backbone sections with alkyl linkers or amino linkers (see, e.g., Kurreck 2003 (Eur J Biochem 270: 1628-1644)). "Alkyl" as used herein particularly encompasses lower hydrocarbon moieties, e.g., C₁-C₄ linear or branched, saturated or unsaturated hydrocarbon, such as methyl, ethyl, ethenyl, propyl, 1-propenyl, 2-propenyl, and isopropyl.

Nucleic acids as intended herein may include naturally occurring nucleosides, modified nucleosides or mixtures thereof. A modified nucleoside may include a modified heterocyclic base, a modified sugar moiety, a modified inter-nucleoside linkage or a combination thereof. The term "nucleic acid" further preferably encompasses DNA, RNA and DNA/RNA hybrid molecules, specifically including hnRNA, pre-mRNA, mRNA, cDNA, genomic DNA, amplification products, oligonucleotides, and synthetic (e.g., chemically synthesised) DNA, RNA or DNA/RNA hybrids. A nucleic acid can be naturally occurring, e.g., present in or isolated from nature, can be recombinant, i.e., produced by recombinant DNA technology, and/or can be, partly or entirely, chemically or biochemically synthesised. A "nucleic acid" can be double-stranded, partly double stranded, or single-stranded. Where single-stranded, the nucleic acid can be the sense strand or the antisense strand. In addition, nucleic acid can be circular or linear.

In the practice of the present invention, the "nucleic acid" in which the presence or absence of the at least one genetic variant or mutation in the *ATP2A1* gene can be determined may particularly preferably refer to DNA, even more preferably to nuclear genomic DNA, or to RNA, in particular RNA transcribed from the *ATP2A1* gene.

The bovine ATPase sarcoplasmic/endoplasmic reticulum Ca2+ transporting 1 (*ATP2A1*) nucleotide sequence may be the sequence corresponding to GenBank Accession No. NM_001075767.1 (also defined herein as SEQ ID NO:1), corresponding to the *ATP2A1* mRNA sequence, or a fragment thereof or a region of the bovine genome that comprises/encodes this mRNA sequence or a fragment thereof.

GAACGGGGGTCAGAGCTTCGTGGAGGGAAGAATAACCTGGGGGGGGGAAGGAAAGAAAAAGGAAGAAACCCAG
ACAAACAGGCAGGTGGACACACTGAGGAAGGCCCCCGACGTGTGAGAACCCCCCCAGAAGGAACACACCGCCC
CCTGGCCCCCAGGAAGGGAGCACCATGGAGGCTGCGCACTCCAAGACCACAGAAGAATGTTTGGCCTATTTTG
GGGTGAGCGAAACTACAGGCCTCACCCCGGACCAAGTTAAGCGGCATCTGGAGAAATATGGCCATAATGAGCT
CCCTGCTGAGGAGGGGAAGTCCCTGTGGGAGCTGGTGTTAGAGCAGTTTGAAGACCTCCTGGTGCGGATCCTT
CTCCTGGCCGCCTGCATTTCCTTTGTGCTGGCCTGGTTTGAGGAAGGCGAAGAGACTGTCACCGCCTTTGTTG
AACCCTTTGTCATCCTCTTGATCCTCATTGCCAATGCCATCGTGGGGGTTTGGCAGGAGCGGAATGCAGAGAA
TGCCATCGAGGCTCTGAAGGAATATGAACCCGAGATGGGGAAGGTCTACCGGGCTGACCGCAAGTCAGTACAA
AGGATCAAAGCTCGGGATATTGTTCCTGGAGACATCGTGGAGGTGGCTGTGGGGGACAAGGTCCCGGCAGATA
TCCGCATCCTCACCATCAAGTCTACCACCCTCCGGGTCGACCAGTCCATCCTGACAGGCGAGTCTGTCTCTGT
CATCAAGCACACAGAACCTGTTCCCGACCCCCGAGCTGTCAACCAGGATAAGAAGAACATGCTTTTCTCGGGC
ACCAACATCGCAGCCGGCAAGGCCATCGGCATTGTGGCCACCACCGGTGTGGGCACCGAGATTGGGAAGATCC
GTGACCAAATGGCCGCCACAGAGCAGGACAAGACCCCTCTGCAGCAAAAGCTGGATGAGTTTGGGGAGCAGCT
CTCCAAGGTCATCTCCCTCATCTGCGTGGCCGTCTGGCTCATCAACATTGGCCACTTCAACGACCCCGTGCAT
GGGGGCTCCTGGATCCGTGGTGCCATCTACTACTTTAAGATCGCCGTGGCCCTGGCTGTGGCTGCCATCCCCG
AGGGCCTTCCTGCAGTCATCACCACCTGCCTGGCCTTGGGCACCCGCCGGATGGCAAAGAAGAACGCGATCGT
GAGGAGCCTGCCCTCCGTGGAGACTCTGGGCTGCACCTCTGTCATCTGTTCCGACAAGACGGGCACCCTCACC
ACCAACCAGATGTCTGTCTGCAAGATGTTCATCATCGACAGGATAGATGGGGACCTCTGCCTGCTGAACGAGT
TCTCTGTCACTGGCTCCACTTACGCTCCTGAAGGAGAGGTCTTGAAGAATGATAAGCCAGTCCGCTCAGGGCA
GTATGACGGGCTGGTGGAGCTGGCCACCATTTGTGCCCTCTGCAATGACTCCTCCTTGGACTTCAATGAGACC
AAAGGTATTTATGAGAAGGTGGGAGAGGCCACCGAGACAGCCCTCACCACCCTGGTGGAGAAGATGAATGTAT
TCAACACTGAAGTGAGAAACCTCTCGAAGGTAGAGAGGGCCAACGCCTGCAACTCGGTGATCCGCCAGCTAAT
GAAGAAGGAATTCACCCTGGAGTTCTCCCGGGACCGAAAGTCCATGTCTGTCTATTGCTCTCCAGCAAAATCC
CGGGCTGCTGTGGGCAATAAGATGTTTGTCAAGGGTGCCCCTGAGGGGGTCATCGATCGCTGTAACTACGTGC
GAGTGGGCACCACCCGGGTGCCCATGACAGGGCCGGTGAAGGAGAAGATTCTGTCGGTGATCAAAGAGTGGGG
TACTGGCCGGGACACCCTGCGCTGCCTGGCGCTGGCCACTCGGGACACCCCCCCCAAGCGAGAGGAGATGGTC
CTGGATGACTCTACCAAGTTCATGGAGTACGAGACGGACTTGACATTCGTTGGCGTCGTGGGCATGCTGGACC
CGCCCCGCAAGGAGGTCATGGGCTCCATCCAACTATGCCGAGACGCTGGAATCCGAGTAATCATGATCACCGG
AGACAACAAGGGCACGGCCATTGCCATCTGCCGACGGATTGGCATCTTCGGGGAGAACGAGGATGTGGCTGAC
CGGGCCTACACTGGCCGGGAGTTCGATGACCTGCCCCTGGCCGAGCAGCGGGAGGCCTGCCGACGCGCCTGCT
GCTTCGCCCGCGTGGAGCCCACCCACAAGTCGAAGATCGTGGAGTATCTGCAGTCCTTCGATGAGATCACGGC
GATGACAGGTGATGGCGTCAATGACGCCCCTGCGCTGAAGAAGGCGGAAATCGGCATTGCCATGGGATCCGGC
ACTGCCGTGGCCAAGACAGCCTCTGAGATGGTGCTGGCCGACGACAACTTCTCCACCATTGTGGCTGCCGTGG
AGGAGGGCCGGGCCATCTACAACAACATGAAGCAGTTTATCCGCTACCTCATCTCCTCCAACGTGGGCGAGGT
GGTCTGCATCTTCCTGACAGCTGCCCTGGGGCTGCCCGAGGCCCTGATCCCTGTGCAGCTGCTGTGGGTGAAC

CTGGTGACCGATGGGCTCCCAGCCACAGCCCTGGGCTTCAACCCCCCAGACCTGGACATCATGGACCGGCCCC
CCCGGACCCCCAAGGAGCCCCTCATCAGTGGCTGGCTCTTCTTCCGCTACATGGCAATTGGGGGCTATGTGGG
TGCAGCCACCGTGGGAGCAGCGGCCTGGTGGTTCCTGTATGCTGAGGACGGGCCTCACGTCACCTACAGCCAG
CTGACTCACTTCATGAAGTGCTCGGAGCACAGCCCAGACTTCGAGGGTGTGGACTGTGAGGTCTTCGAGGCCC
CGCAGCCCATGACCATGGCCTTGTCTGTGCTGGTCACCATCGAGATGTGCAATGCTCTCAACAGCCTGTCTGA
GAATCAGTCCCTCGTGCGGATGCCACCCTGGGTGAACATCTGGCTGGTGGGCTCCATCGGCCTCTCCATGTCC
CTCCACTTCCTCATCCTCTACGTGGACCCTCTGCCGATGATCTTCAAGCTCCAAGCCCTGGACCTGTACCATT
GGCTCATGGTCCTCAAGATCTCATTACCAGTCATTGGGCTCGACGAAATCCTCAAGTTCGTTGCTCGAAACTA
CCTGGAGGGATAACCCGTTTCCCCCTCCTCCATCTCTTTGAACCGTGTCACAGATCCAGAAGATGAAAGAAGG
AAGCAACCAGGCCTTTTGCCCTCCCTCCCCACCCCATAGTGACGCATCTTCGGCCAGAGCTGTGGCACAGGCC
CCACCCTCCACAACGTTCCCTATTCCTCCCGCCCGACATTCTTGTTTATAAACACTATGTCCCCTTCACTTCT
CCTCCCCCCTCAGCAACCTGTCCCCCTTTTGCCCTTGTGAAACCTCCCCTCCCCGACCCCGTGGGGCTCGCAG
GGACAAGGCGACTGATTGAGCTGAGCTGCTTATTTATTGAAAATAAATGACAGAAAAATCAAAAAAAAAAAAA
AAAAAAAAAAAA (SEQ ID NO:1)

The corresponding amino acid sequence may be the sequence corresponding to NCBI Accession No. NP_001069235.1 also defined herein as SEQ ID NO:2, and as depicted hereafter:

MEAAHSKTTEECLAYFGVSETTGLTPDQVKRHLEKYGHNELPAEEGKSLWELVLEQFEDLLVRILLLAAC
ISFVLAWFEEGEETVTAFVEPFVILLILIANAIVGVWQERNAENAIEALKEYEPEMGKVYRADRKSVQRI
KARDIVPGDIVEVAVGDKVPADIRILTIKSTTLRVDQSILTGESVSVIKHTEPVPDPRAVNQDKKNMLFS
GTNIAAGKAIGIVATTGVGTEIGKIRDQMAATEQDKTPLQQKLDEFGEQLSKVISLICVAVWLINIGHFN
DPVHGGSWIRGAIYYFKIAVALAVAAIPEGLPAVITTCLALGTRRMAKKNAIVRSLPSVETLGCTSVICS
DKTGTLTTNQMSVCKMFIIDRIDGDLCLLNEFSVTGSTYAPEGEVLKNDKPVRSGQYDGLVELATICALC
NDSSLDFNETKGIYEKVGEATETALTTLVEKMNVFNTEVRNLSKVERANACNSVIRQLMKKEFTLEFSRD
RKSMSVYCSPAKSRAAVGNKMFVKGAPEGVIDRCNYVRVGTTRVPMTGPVKEKILSVIKEWGTGRDTLRC
LALATRDTPPKREEMVLDDSTKFMEYETDLTFVGVVGMLDPPRKEVMGSIQLCRDAGIRVIMITGDNKGT
AIAICRRIGIFGENEDVADRAYTGREFDDLPLAEQREACRRACCFARVEPTHKSKIVEYLQSFDEITAMT
GDGVNDAPALKKAEIGIAMGSGTAVAKTASEMVLADDNFSTIVAAVEEGRAIYNNMKQFIRYLISSNVGE
VVCIFLTAALGLPEALIPVQLLWVNLVTDGLPATALGFNPPDLDIMDRPPRTPKEPLISGWLFFRYMAIG
GYVGAATVGAAAWWFLYAEDGPHVTYSQLTHFMKCSEHSPDFEGVDCEVFEAPQPMTMALSVLVTIEMCN
ALNSLSENQSLVRMPPWVNIWLVGSIGLSMSLHFLILYVDPLPMIFKLQALDLYHWLMVLKISLPVIGLD
EILKFVARNYLEG (SEQ ID NO:2).

Amino acid residues that may be substituted in bovines carrying at least one genetic variant or mutation in the *ATP2A1* gene as described herein are underlined.

When reference is made herein to the ATP2A1 protein, the ATP2A1 protein of SEQ ID NO:2 may be denoted.

In a first embodiment, a method is provided for determining the genotype of a bovine for DMS-causing *ATP2A1* variants by analyzing its genomic DNA, the method comprising the steps of:
a. obtaining a sample of material containing said genomic DNA from the bovine,
b. extracting the DNA from said sample,
c. genotyping said DNA for one or more DMS-causing *ATP2A1* variants, and
d. determining whether said animal is homozygous, compound heterozygous or heterozygous for DMS-causing *ATP2A1* variants.

The term "bovine" in accordance with the present invention encompasses all cattle or cattle breeds from the species *Bos taurus.* In a preferred embodiment of the methods of the present invention the bovine is selected from the group consisting of Belgian Blue Cattle, Charolais, Roodbont, Piedmontese, Chianina or Romagnola.

The term "sample" or "biological sample" according to the present invention refers to any material containing a nucleic acid, preferably nuclear DNA and/or RNA, from said bovine to be tested. In a preferred embodiment the biological sample to be used in the methods of the present invention is selected from the group consisting of blood, sperm, hair roots, milk, body fluids as well as tissues including nucleated cells.

DNA extraction / isolation and purification methods are well-known in the art and can be applied in the present invention. Standard protocols for the isolation of genomic DNA are inter alia referred to in Sambrook, J., Russell, D.W., Molecular Cloning: A Laboratory Manual, the third edition, Cold Spring Harbor Laboratory Press, Cold Spring Harbor, New York, 1.31-1.38, 2001 and Sharma, R.C., et al., A rapid procedure for isolation of RNA-free genomic DNA from mammalian cells, BioTechniques, 14, 176-178, 1993.

In a preferred embodiment of the invention the term "DMS-causing *ATP2A1* variants" refers to *R143W ATP2A1* mutation reported in BBC and Charolais. In another embodiment of the invention, it refers to the R559C *ATP2A1* mutation reported in BBC, the R164H *ATP2A1* mutation, the G211V *ATP2A1* mutation, the G286V *ATP2A1* mutation, the G284V *ATP2A1* mutation, or the *ATP2A1* mutation reported in Piemontese, or the *ATP2A1* mutation reported in Romagnola, or the *ATP2A1* mutation reported in Chianina, or the *ATP2A1* mutation reported in Roodbont. In yet another embodiment of the mutation it refers to naturally occurring *ATP2A1* variants or mutations that encode either loss-of-function or hypomorphic ATP2A1 variants or mutants. As used herein a "*R143W ATP2A1* variant or mutation" and the like refers to an *ATP2A1* genetic variant or mutation leading to a R143W substitution in the ATP2A1 protein, in particular the ATP2A1 of SEQ ID NO:2.

The term "genotyping" generally refers to a method for determining or identifying whether a particular nucleotide sequence (also referred to as a genetic marker) is present or absent at one or more particular positions within the genome of a subject. Preferably, such methods involve the use of a nucleic acid such as DNA or RNA from the bovine. Also envisaged herein includes assaying for protein conformational or sequences changes which occur in the presence of an *ATP2A1* genetic variant or mutation. The *ATP2A1* genetic variant or mutation may or may not be the causative mutation but will be indicative of the presence of this change and one may assay for the genetic or protein bases for the phenotypic difference.

The term "genotyping said DNA for one or more DMS-causing *ATP2A1* variants" in accordance with the present invention refers to a method for determining or identifying whether a particular nucleotide sequence is present in a DNA sample.

There are several methods known by those skilled in the art, e.g. (7) for determining whether such nucleotide sequence is present in a DNA sample. These include the amplification of a DNA segment encompassing the mutation by means of the polymerase chain reaction (PCR) or any other amplification method, and interrogate the amplicons by means of allele specific hybridization, or the 3'exonuclease assay (Taqman assay), or fluorescent dye and quenching agent-based PCR assay, or the use of allele-specific restriction enzymes (RFLP-based techniques), or direct sequencing, or the oligonucleotide ligation assay (OLA), or pyrosequencing, or the invader assay, or minisequencing, or DHPLC-based techniques, or single strand conformational polymorphism (SSCP), or allele-specific PCR, or denaturating gradient gel electrophoresis (DGGE), temperature gradient gel electrophoresis (TGGE), chemical mismatch cleavage (CMC), heteroduplex analysis based system, techniques based on mass spectroscopy, invasive cleavage assay, polymorphism ratio sequencing (PRS), microarrays, a rolling circle extension assay, HPLC-based techniques, extension based assays, ARMS (Amplification Refractory Mutation System), ALEX (Amplification Refractory Mutation Linear Extension), SBCE (Single base chain extension), a molecular beacon assay, invader (Third wave technologies), a ligase chain reaction assay, 5'-nuclease assay-based techniques, hybridization capillary array electrophoresis (CAE), protein truncation assay (PTT), immunoassays and solid phase hybridization (dot blot, reverse dot blot, chips). This list of methods is not meant to be exclusive, but just to illustrate the diversity of available methods. In embodiments, Illumina SNP array is used. In embodiments, Taqman amplification is used.

Some of these methods can be performed in accordance with the methods of the present invention in microarray format (microchips) or on beads.

The invention thus also relates to the use of primers or primer pairs, wherein the primers or primer pairs hybridize(s) under stringent conditions to the DNA corresponding to the bovine *ATP2A1* gene, or to the complementary strand thereof. Preferably, the primers of the invention have a length of at least 14 nucleotides such as 17 or 21 nucleotides.

In alternative embodiments, the nucleic acid such as the DNA may be sequenced for one or more DMS-causing *ATP2A1* variants or mutations. There are many methods known in the art for determining the sequence of a nucleic acid such as DNA in a sample, such as Sanger sequencing, and various approaches of massively parallel sequencing (next generation sequencing, NGS), and for identifying whether a given nucleic acid sample contains a particular nucleotide sequence. Any such technique known in the art may be used in performance of the methods of the present invention.

In particular embodiments, the presence or absence of an *ATP2A1* genetic variant or mutation in the *ATP2A1* gene may be determined by sequencing at least a portion of the *ATP2A1* gene, such as a portion of the *AZP2A1* gene that spans the location of the *ATP2A1* genetic variant or mutation. Many methods of sequencing genomic DNA are known in the art, and any such method can be used, see for example Sambrook et al. Molecular Cloning: A Laboratory Manual 2d ed. (1989). For example, a genomic DNA fragment spanning the location of the *ATP2A1* genetic variant or mutation of interest can be amplified using the polymerase chain reaction. The amplified region of DNA form can then be sequenced using any method known in the art, for example using an automatic nucleic acid sequencer. The detection of a given *ATP 2AI* genetic variant or mutation can then be performed using hybridization of probes or using PCR-based amplification methods as known to the skilled person.

In a further embodiment of the present invention a method is provided for determining the genotype of a bovine for DMS-causing *ATP2A1* variants by analyzing its RNA, the method comprising the steps of:
a. obtaining a sample of material containing said RNA from the bovine,
b. extracting the RNA from said sample,
c. genotyping said RNA for one or more DMS-causing *ATP2A1* variants, and
d. determining whether said animal is homozygous, compound heterozygous or heterozygous for DMS-causing *ATP2A1* variants.

"RNA" as referred to herein encompasses all types of RNA. Techniques well known in the art in order to allow for the isolation of total RNA, mitochondrial RNA, or messenger RNA. The person skilled in the art can select a suitable extraction method without further ado depending on the nature of the sample to be tested.

If a sample containing RNA is to be used as a template for an amplification reaction, it will be necessary to transcribe said RNA in cDNA before amplification can be carried out. Again, techniques for doing so are well known to the person skilled in the art. As an example the RNA may be purified with RNeasy^{™} Mini Kit (Qiagen). The RNA will then be reversely transcribed to cDNA using, e.g. the SuperScript^{™} Choice System (Invitrogen).

In embodiments, said at least one *ATP2A1* genetic variant or mutation comprises a hypomorphic mutation in the *ATP2A1* gene. As used herein, a "hypomorphic" mutation refers to a mutation that causes a partial loss of gene function, i.e. a reduction in gene function e.g. through reduced (protein, RNA) expression or reduced functional performance, but preferably not a complete loss.

In particular embodiments, the *ATP2A1* genetic variant or mutation or DMS-causing *ATP2A1* variant is a single nucleotide polymorphism (SNP) or a point mutation.

A "single nucleotide polymorphism" or "SNP", and "a point mutation" refer to a variation in the nucleotide sequence of a polynucleotide that differs from another polynucleotide by a single nucleotide difference. For example, without limitation, exchanging one A for one C, G or T in the entire sequence of polynucleotide constitutes a SNP or point mutation. Of course, it is possible to have more than one SNP or point mutation in a particular polynucleotide. For example, at one position in a polynucleotide, a C may be exchanged for a T, at another position a G may be exchanged for an A and so on. When referring to SNPs or point mutations, the polynucleotide is most often DNA. If a point mutation is occurring frequently (e.g. frequency is higher than 1%) in a given population, it may be denoted a "SNP".

In further embodiments, the method further comprises determining whether the bovine is heterozygous, compound heterozygous, or homozygous for the at least one *ATP2A1* genetic variant or mutation.

By "homozygous" or "homozygous polymorphism" is meant that the two alleles of a diploid cell or organism at a given locus are identical, that is, that they have the same nucleotide for nucleotide exchange at the same place in their sequences. By "heterozygous" or "heterozygous polymorphism" is meant that the two alleles of a diploid cell or organism at a given locus are different, that is, that they have at least one different nucleotide exchanged for the same nucleotide at the same place in their sequences. By "compound heterozygous" is meant herein that the two alleles of a diploid cell or organism at a given locus are different, that is, that they have two different nucleotides exchanged for the same nucleotide at the same place in their sequences.

In embodiments, the heterozygous or homozygous presence of the at least one *ATP2A1* genetic variant or mutation, or the DMS-causing ATP2A1 variant, is indicative of the bovine having or being susceptible to TDMS.

As used herein, the term "locus" or "loci" refers to the site of a gene on a chromosome. Pairs of genes, known as "alleles" control the hereditary trait produced by a gene locus. Each animal's particular combination of alleles is referred to as its "genotype". Where both alleles are identical the individual is said to be homozygous for the trait controlled by that gene pair; where the alleles are different, the individual is said to be heterozygous for the trait.

A further aspect is directed to a method for selecting a bovine animal for meat quality using a method described herein. As noted above, a bovine is affected by or susceptible to Tough and Dry Meat Syndrome (TDMS) is more likely to yield meat of inferior meat quality. Further, the genetic markers as described herein have been shown to be associated with meat quality in younger bovines, both males and females. Thus, the present invention provides methods for screening bovines to identify those more likely to produce favorable meat quality, and/or those less likely to produce favorable meat quality to optimize breeding and selection techniques for the best meat quality.

In embodiments, the heterozygous or homozygous presence of the at least one *ATP2A1* genetic variant or mutation is indicative for the bovine animal to possess inferior meat quality.If a bovine tests positive for the at least one *ATP2A1* genetic variant or mutation, such animal can be culled from the group and otherwise used. If the animal does not test positive for the at least one *ATP2A1* genetic variant or mutation the animal can be considered for inclusion in the breeding program. Use of this genotype data can also be incorporated with the screening for multiple alleles for meat quality.

The present invention will now be further illustrated by means of the following non-limiting examples.

### Examples

### Example 1: DMS is caused by the R143W mutation in the ATP2A1 gene

We obtained meat samples from 38 animals suspected of DMS. From those we extracted total genomic DNA, which we genotyped with a SNP array interrogating 9,457 random SNPs, as well as 1,550 coding variants that were identified in whole genome and whole exome data of ~80 Belgian Blue bulls (Charlier et al., 2016). We used available genotype data from 1,058 unrelated, age-matched Belgian Blue animals, representative of the general Belgian Blue Cattle (BBC) population, as controls. We conducted a GWAS, tested the association of (i) individual SNPs, and (ii) haplotype-based hidden states (HS) with phenotype using a generalized linear mixed model (GLMM) with the logit link function (Zhang et al., 2012). To mitigate stratification, we included a random polygenic effect estimated using a SNP or haplotype-based genomic relationship matrix G (Zhang et al., 2012). We obtained a single genome-wide significant signal on chromosome 25 with the two models (single SNP and 10 HS) (Fig. 1). The most significant single SNP was a missense variant (*R143W*) in the *ATP2A1* gene encoding the SERCA1 protein (p = 3.6×10⁻²¹). The maximum 10 HS signal (p ≤ 6.2×10⁻²³) overlapped with this variant, and was due to the enrichment in cases of a single haplotype that was in near-perfect linkage disequilibrium (*r*² ≥ 0.91) with the *ATP2A1 R143W* variant. *R143W* had a frequency of 15% in the general BBC population, yet of 55% in the 38 affected samples (p = 9.5×10⁻²³) (Fig. 1).

We locally (1Mb window centered on the *ATP2A1 R143W* variant) imputed all animals to whole genome using genome-wide sequence data from BBC bulls as reference population, and repeated the single SNP association analyses. The *ATP2A1 R143W* variant remained the most strongly associated variant (data not shown).

The SERCA1 protein is a Ca²⁺ ATPase that is embedded in the membrane of the sarcoplasmic reticulum of muscle fibers, pumping Ca²⁺ ions from the sarcoplasm back into the reticulum thereby controlling muscle relaxation (Wuytack at al., 2002). Loss-of-function mutations in *ATP2A1* cause autosomal recessive Brody myopathy in humans (Brody, 1969; Odermatt et al., 1996&2000; OMIM 601003), Congenital Muscular Dystonia I in Belgian Blue Cattle (*R559C*; Charlier et al., 2008; OMIA 001464-9913), and perinatal lethality from impaired diaphragm function in *ATP2A1* knockout mice (Pan et al., 2003). Missense *ATP2A1* variants have been associated with recessive myotonia of variable severity in at least four other beef cattle breeds (OMIA 001464-9913): Chianina (Drögemüller et al., 2008), Piedmontese (Grünberg et al., 2010), Romagnola (Murgiano et al., 2012), Roodbont (Grünberg et al., 2010; Dorotea et al. 2015).

The *R143W*mutation was located in *ATP2A1* coding exon 5 and affected a highly conserved residue in the first cytoplasmic loop of SERCA1 (Wuytack at al., 2002) (Fig. 2). Taken together our results demonstrate that *143W* is a hypomorphic *ATP2A1* variant that causes DMS.

### Example 2: In depth characterization of the DMS phenotype

We performed a systematic survey for "tough and dry" meat cuts in a Belgian slaughter-plant in Anderlecht between November 2019 and September 2020. Out of a first series of 496 examined carcasses of adult Belgian Blue cows, 62 (= 12.5%) were considered to be affected to various extend. The first striking feature was the systematic increased loss of fluid from the carcass (referred to as "drip") during the cooling process (i.e. the period between slaughter and cutting). Upon cutting of the carcasses, affected animals exhibited exudate at the surface of the *m. semimembranosus,* and a "dry and tough" feeling of the *m. adductor femoris* and inner parts of the *m. glutaeobiceps* and *m. semimembranosus,* as well as of the *m. quadriceps femoris, m. semitendinosus, m. longissimus dorsi, m. subscapularis* and *m. teres major* for the most severe cases (Fig. 3). Animals were given a DMS severity score between 0 and 3 (0: unaffected; 1-1.5: exudate at the surface of the *m. semimembranosus,* "dry and tough" feeling of the *m. adductor femoris* and inner parts of the *m. glutaeobiceps* and *m. semimembranosus;* 2-2.5-3: same as before, plus "dry and tough" feeling of the *m. quadriceps femoris, m. semitendinosus, m. longissimus dorsi, m. subscapularis* and *m. teres major*).

Samples of the *m. glutaeobiceps* were collected from 26 affected (5 x category 1; 9 x 1.5; 9 x 2; 3 x 2.5) and 21 non-affected animals and analyzed for standard meat quality traits (Table 1). Meat samples of affected cows had altered color (darker) and color stability, texture (higher hardness, gumminess, chewiness, and shear force), and moisture content (reduced EZ-drip, thawing and cooking loss; increased dry matter content). There was no effect on pH or protein solubility.

**Table 1: Meat quality of dry and normal m. glutaeobiceps samples**

| | **'Dry'** | **Normal** | **p-value** |
|---|---|---|---|
| **Number of cases** | **26** | **21** | |
| Age at slaughter (month) | **54.6** ± 16.3 | **57.1** ± 14.9 | NS |
| Carcass weight (kg) | **536** ± 53 | **540** ± 45 | NS |
| pHᵤ | **5.72** ± 0.06 | **5.68** ± 0.10 | NS |
| CIE L* (lightness) | **43.0** ± 3.9 | **46.1** ± 2.9 | 0.003 |
| CIE a* (redness) | **22.4** ± 1.7 | **25.1** ± 1.9 | <0.001 |
| CIE b* (yellowness) | **21.7** ± 2.5 | **26.6** ± 1.4 | <0.001 |
| CIE Δa* (d0-7) | **16.5** ± 2.7 | **19.1** ± 2.5 | 0.002 |
| TPA - Hardness (N) | **40.7** ± 8.3 | **29.5** ± 8.2 | <0.001 |
| TPA - Cohesiviness | **0.40** ± 0.05 | **0.39** ± 0.06 | NS |
| TPA - Adhesiviness (N) | **-0.71** ± 0.89 | **-0.35** ± 0.71 | NS |
| TPA - Springiness | **0.68** ± 0.05 | **0.71** ± 0.07 | NS |
| TPA - Gumminess (N) | **16.0** ± 3.6 | **11.3** ± 3.0 | <0.001 |
| TPA - Chewiness (N) | **10.9** ± 2.8 | **7.9** ± 2.1 | <0.001 |
| EZ-Driploss (%) | **2.11** ± 1.24 | **2.71** ± 1.30 | NS |
| Thawing loss (%) | **7.19** ± 1.45 | **8.42** ± 1.28 | 0.004 |
| Cooking loss (%) | **26.5** ± 1.7 | **28.3** ± 1.7 | 0.001 |
| Dry matter (%) | **27.4** ± 0.7 | **26.6** ± 0.7 | <0.001 |
| WB Shear force (N) | **55.7** ± 2.2 | **37.7** ± 4.8 | <0.001 |
| Sarcomere length (µm) | **1.40** ± 0.14 | **1.51** ± 0.16 | 0.018 |
| Solubility sarcoplasmatic protein fraction (mg/g meat) | **48.9** ± 4.5 | **50.0** ± 4.5 | NS |
| Solubility myofibrillar protein fraction (mg/g meat) | **19.4** ± 1.9 | **19.3** ± 2.6 | NS |
| NS (p>0.05) | | | |

The affected ('dry') and non-affected ('normal') animals had no difference in age (54.6 vs. 57.1 months) and carcass weight (536 vs. 540 kg). The color of dry meat appeared significantly darker (L*=43.0 instead of 46.1), less red (a*=22.4 vs. 25.1) and less yellow (b*=21.7 vs. 26.6). After storage for 7 days under light, the a*-value of both ended at the same level (5.97 vs. 5.93). The ultimate pH was almost the same in both groups (5.72 vs. 5.68), marking a clear difference between this described phenomenon and DFD-meat. The 'dry' feeling of the dry meat was confirmed with a significantly higher dry matter content (27.4% vs. 26.6%) and lower thaw and cooking losses (7.19% vs. 8.42% and 26.5% vs. 28.3%), despite the non-significant difference in drip loss (2.11% vs. 2.71%). The 'tough' feeling of the dry meat was confirmed by the significantly higher hardness (40.7N vs. 29.5N) and higher shear force values (55.7N vs. 37.7N). The difference in these texture properties could partially be explained by the significantly shorter sarcomere length of dry meat (1.40µm vs. 1.51µm) as a direct result of distorted fiber relaxations caused by the mutation in the *ATP2A1* gene. However, no explanation for the dryness could be provided, with no differences in sarcoplasmatic and myofibrillar protein solubility used as indicator for protein denaturation.

Referring to the anomaly as "tough and dry meat" may at first glance appear to contradict the primary manifestation of the condition, namely the marked increase in fluid loss from the carcass during the cooling period, as well as the observation of deep exudates upon section (Fig. 3). However, it is most likely this early loss of fluids (between slaughter and cutting) that causes the subsequent dryness (increased dry matter content) and apparent improved water-holding-capacity of the meat. It can be calculated that the observed difference in dry matter content of 0.8% corresponds to increased fluid losses of 2.9% in the dry meat samples, limiting the amount of fluid that can be further lost during subsequent handling and storage. The sensory quality trait that is most affected, is tenderness. There is a marked increase in WB shear force and instrumentally measured hardness, likely related to the shorter sarcomere length. The average difference in WB shear force of 18 N will definitely result in lower scores for tenderness by an expert or consumer panel.

### Example 3: Estimating the penetrance of DMS in R143W hetero- and homozygous cows.

We genotyped DNA extracted from 116 affected samples (62 from the series described above in Example 2 and 54 from a second series) as well as 127 unaffected control samples collected in the same slaughter-plant and during the same period as described in Example 2 using the bovine Illumina medium density (MD) genotyping array following the manufacturer's instructions.

A case-versus-control GWAS confirmed the chromosome 25 association maximizing at the *ATP2A1 R143W* position with individual SNP (p = 1.7×10⁻³⁶) and the HS10 (p = 4.8×10⁻⁵⁴) models (Fig. 4). The frequency of the *R143W* allele was 8.2% in the "hyper" controls (without a single homozygote *W*/*W* animal), while it was 55.1% in cases (without a single homozygote R/R animal). We further tested (within cases) whether there was an association between *R143W* genotype and severity of the condition by regressing dry meat score (categories 1 to 3) on dosage of the W allele (1 or 2). The average score of *W*/*R* cases was 1.46 while it was 1.92 for *W*/*W* cases (p = 0.0005) (Fig. 4).

Assuming that the prevalence of the condition is 12.5% in the BBC population (see above) and given the observed genotypic proportions observed in cases and controls (Fig. 1), we calculated that the penetrance of the anomaly is 43% in heterozygotes and 100% in homozygotes.

In an initial attempt to identify potential modifiers of the *R143W* mutation, we performed a GWAS for the severity of the condition (categories 0 to 3) in the 122 W/R animals only. This didn't yield any genome-wide significant signal (data not shown).

Using available genotypes from > 65,000 Belgian Blue animals born between 2014 and 2020, we retrospectively examined the change in frequency of the *R143W* variant in the general BBC population over time. It showed a clear linear upward trend from 14.5% in 2014 to 17.5% in 2020.

### Example 4: Evaluating the economic impact of the R143W ATP2A1 mutation in BBC

Heterozygotes and homozygotes at present account for 30% and 3%, respectively, of a cow population of 500,000 of which 140,000 are slaughtered each year.

We can estimate the depreciation of the carcasses by assuming that the affected cuts can only be valorized as minced meat. Depreciation is then calculated by multiplying the difference in average market price of the different cuts with the price of minced meat, and their corresponding net weight (average of the weight of the cuts of 10 Belgian Blue carcasses). The value loss of a carcass with dry meat in category 1 was estimated at 13%, for a carcass in category 2 and 3 this value loss increased to 23% and 27% respectively.

From the 62 affected animals analyzed between January and March 2020, 27% were classified in category 1, 45% in category 1.5, 23% in category 2 and 5% in category 2.5. No case of category 3 was observed during these months. Hence, the average depreciation of affected carcasses can be estimated at roughly 16% which at present equates to ~560 €.

Combined with the observed genotype-specific frequencies and penetrances and assuming that all affected carcasses are detected and equally depreciated, this amounts to an estimated annual loss of ∼ 13,000,000€ for the entire Belgian BBC cow population.

Thus far, the anomaly has only been reported and investigated in cow carcasses. Half of the BBC animals slaughtered each year are young bulls. This suggests that either age and/or sex and/or fattening regime affect the penetrance of the condition. Further work is needed to determine which factors affect the penetrance of the *R143W ATP2A1* mutation, and whether the carcass quality of younger animals is also affected albeit in a subtler manner.

### Example 5: DNA testing against the R143W mutation

Knowing the causative mutation will allow for effective counter selection against the *R143W* variant. As heterozygotes may have a probability as high as ~40% to be affected, avoiding matings between carrier animals is not sufficient to control the condition. Animals carrying the *R143W* mutation should be identified e.g. by genotyping and progressively eliminated from the breeding stock.

One way to achieve this is to incorporate the effect of *R143W* genotype on carcass worth in the breeding goal with the other selected phenotypes.

Care will have to be taken to also properly account for inbreeding depression that may accompany a too rapid elimination of the *R143W* variant that now has a frequency approaching 18% in the general BBC population. Of note, *R143W* has also been reported in Charolais.

### Example 6: DNA testing against loss-of-function or hypomorphic ATP2A1 mutations other than R143W

*ATP2A1* coding variants other than *R143W*have been reported in several cattle breeds and have been found to cause recessive Congenital Muscular Dystonia-I (CMD-1) in BBC and myotonia of variable severity in other cattle breeds (i.e. only homozygous animals are affected).

It was tested whether these mutations also cause DMS in these cattle breeds (especially in the most heavily muscled ones), including in heterozygotes. We examined the frequency of the *R559C ATP2A1* mutation (causing CMD-I in homozygotes) in the initial series of 38 DMS samples (the *R559C* mutation has been eliminated from the BBC population by DNA testing and was therefore absent in the second data series). Three of the 38 samples were compound *R559C*/*R143W* heterozygotes and one was *R559*/+ heterozygote, corresponding to a frequency of 5.2%. The frequencies of these two genotypes were significantly higher (Fisher's exact test, conditional on the effect of the R143W mutation: p = 0.000142) than expected by chance alone, showing that the *R559C* mutation contributes to DMS as well.

### Example 7: The R143W ATP2A1 mutation also affects meat quality in younger animals, both males and females.

The effect of the *R143W ATP2A1* mutation on meat quality, and the fact that it causes DMS, was first uncovered in elder cows, which account for approximately halve of the slaughtered animals in Belgian Blue. The other halve are younger animals, including both males and females.

To examine whether the *R143W* mutation also affects meat quality at a younger age, and hence in essence in all animals at slaughter, the slaughtering procedure of 152 Belgian Blue animals (106 young males and 46 females) in two slaughterhouses, in Jupille (Leemput) and Aubel, was followed. The animals were classified, by visual examination of the carcasses, in three categories: SUSPECT, UNDETERMINED, GOOD. Meat samples from the corresponding carcasses were collected and used to extract DNA and genotype the corresponding animals for the *R143W* mutation. Table 2 summarizes the observed results.

**Table 2. Evaluation of the effect of the R143W ATP2A1 mutation on meat quality in young animals.**

| | R143W genotype | SUSP | UNDET | GOOD |
|---|---|---|---|---|
| Jupille, male | W/W | 0 | 1 | 0 |
| | R/W | 17 | 10 | 3 |
| | R/R | 12 | 22 | 28 |
| Jupille, female | W/W | 1 | 0 | 0 |
| | R/W | 10 | 6 | 1 |
| | R/R | 3 | 5 | 8 |
| Aubel, male | W/W | 1 | 0 | 0 |
| | R/W | 4 | 0 | 1 |
| | R/R | 2 | 1 | 4 |
| Aubel, female | W/W | 0 | 0 | 0 |
| | R/W | 4 | 0 | 1 |
| | R/R | 3 | 1 | 3 |

In both males and females, the proportion of animals classified as SUSPECT was higher in R/W heterozygote carriers of the *R143W ATP2A1* mutation when compared to the homozygous "wild-type" R/R animals (pₘₐₗₑₛ = 0.0075; p_{females} = 0.021). Although the number of samples collected in Aubel was limited, the trend was the same as in the Jupille slaughter-plant. Three animals were homozygote W/W. Two of these were classified as SUPSECT and one as UNDETERMINED. Taken together, these results support an effect of the *R143W ATP2A1* mutation on meat quality in young animals as well.

### References

Anonymous (1981). Classification scheme for beef carcass classes. Official Journal of the European Communities. Council Regulations No. 1208/81 and 2930/81.
Brody IA (1969). Muscle contracture induced by exercise: a syndrome attributable to decreased relaxing factor. New Eng. J. Med. 281: 187-192.
Browning SR and Browning BL (2007). Rapid and accurate haplotype phasing and missing-data inference for whole-genome association studies by use of localized haplotype clustering. Am. J. Hum. Genet. 81, 1084-1097.
Charlier C, Coppieters W, Rollin F, Desmecht D, Agerholm JS, Cambisano N, Carta E, Dardano S, Dive M, Fasquelle C, Frennet JC, Hanset R, Hubin X, Jorgensen C, Karim L, Kent M, Harvey K, Pearce BR, Simon P, Tama N, Nie H, Vandeputte S, Lien S, Longeri M, Fredholm M, Harvey RJ, Georges M (2008). Highly effective SNP-based association mapping and management of recessive defects in livestock. Nature Genetics 40:449-454.
Charlier C, Li W, Harland C, Littlejohn M, Coppieters W, Creagh F, Druet T, Faux P, Guillaume F, Karim L, Keehan M, Davis S, Kumar-Kadri N, Spelman R, Georges M (2016). NGS-based reverse genetic screen for embryonic lethal mutations compromising fertility in livestock. Genome Res 26: 1333-1341.
Dorotea T, Grünberg W, Murgiano L, Plattet P, Drögemüller C, Mascarello F , Sacchetto R (2015). Fast-twitch skeletal muscle fiber adaptation to SERCA1 deficiency in a Dutch Improved Red and White calf pseudomyotonia case. Neuromuscul Disord 25:888-97.
Drögemüller C, Drögemüller M, Leeb T, Mascarello F, Testoni S, Rossi M, Gentile A, Damiani E, Sacchetto R (2008). Identification of a missense mutation in the bovine ATP2A1 gene in congenital pseudomyotonia of Chianina cattle: an animal model of human Brody disease. Genomics 92:474-7.
Druet T. and Georges M (2010). A hidden markov model combining linkage and linkage disequilibrium information for haplotype reconstruction and quantitative trait locus fine mapping. Genetics 184, 789-798.
Fasquelle et al., 2009 (balancing selection MRC2)
Fujii, J. et al. (1991) Identification of a mutation in the porcine ryanodine receptor associated with malignant hyperthermia. Science 253, 448-451.
Georges M, Charlier C, Hayes B (2019)
Grünberg W, Sacchetto R, Wijnberg I, Neijenhuis K, Mascarello F, Damiani E, Drögemüller C (2010). Pseudomyotonia, a muscle function disorder associated with an inherited ATP2A1 (SERCA1) defect in a Dutch Improved Red and White cross-breed calf. Neuromuscul Disord. 20:467-70.
Gualdron Duarte JL, Gori AS, Hubin X, Lourenco D, Charlier C, Misztal I, Druet T (2020). Performances of Adaptive MultiBLUP, Bayesian regressions, and weighted-GBLUP approaches for genomic predictions in Belgian Blue beef cattle. BMC Genomics 21:545.
Ioannidis JP, Thomas G, Daly MJ (2009). Validating, augmenting and refining genome-wide association signals. Nat Rev Genet., 10(5):318-29.
Miklan et al. [RN]
Murgiano L, Sacchetto R, Testoni S, Dorotea T, Mascarello F, Liguori R, Gentile A, Drögemüller C (2012). Pseudomyotonia in Romagnola cattle caused by novel ATP2A1 mutations. BMC Vet Res 8:186. Romagnola
Odermatt A, Taschner PEM, Khanna VK, Busch HFM, Karpati G, Jablecki CK, Breuning MH, MacLennan DH (1996). Mutations in the gene-encoding SERCA1, the fast-twitch skeletal muscle sarcoplasmic reticulum Ca(2+) ATPase, are associated with Brody disease. Nature Genet. 14: 191-194.
Odermatt A, Barton K, Khanna, VK, Mathieu J, Escolar D, Kuntzer T, Karpati G, MacLennan DH (2000) The mutation of pro(789) to leu reduces the activity of the fast-twitch skeletal muscle sarco(endo)plasmic reticulum Ca(2+) ATPase (SERCA1) and is associated with Brody disease. Hum. Genet. 106: 482-491.
Ott J. (1999). Analysis of Human Genetic Linkage. Third edition. Johns Hopkings University Press.
Pan Y, Zvaritch E, Tupling AR, Rice WJ, de Leon S, Rudnicki M, McKerlie C, Banwell BL, MacLennan DH (2003). Targeted disruption of the ATP2A1 gene encoding the sarco(endo)plasmic reticulum Ca2+ ATPase isoform 1 (SERCA1) impairs diaphragm function and is lethal in neonatal mice. J Biol Chem. 278:13367-75.
Sartelet et al., 2012 a, b (balancing selection, MRC2 and RNF11)
Wuytack F, Raeymaekers L, Missiaen L (2002). Molecular physiology of the SERCA and SPCA pumps. Cell Calcium 32:279-305.
Zhang Z, Guillaume F, Sartelet A, Charlier C, Georges M, Farnir F, Druet T. (2012) Ancestral haplotype-based association mapping with generalized linear mixed models accounting for stratification. Bioinformatics;28(19):2467-73

## Claims

1. A method for determining whether a bovine is affected by or susceptible to Tough and Dry Meat Syndrome (TDMS), comprising determining the presence or absence of at least one genetic variant or mutation in the *ATPZA1* gene in a nucleic acid from the bovine.

2. The method according to claim 1, wherein the presence or absence of the at least one *ATP2A1* genetic variant or mutation is determined by genotyping or sequencing of at least a portion of the *ATP2A1* gene.

3. The method according to claim 1 or 2, wherein said at least one *ATP2A1* genetic variant or mutation comprises a hypomorphic mutation in the *ATP2A1* gene.

4. The method according to any one of claims 1 to 3, wherein said at least one *ATP2A1* genetic variant or mutation comprises one or more variant or mutation selected from the group consisting of: a variant or mutation leading to a substitution at position 143 of the ATP2A1 protein, a variant or mutation leading to a substitution at position 559 of the ATP2A1 protein, a variant or mutation leading to a substitution at position 164 of the ATP2A1 protein, a variant or mutation leading to a substitution at position 211 of the ATP2A1 protein, a variant or mutation leading to a substitution at position 286 of the ATP2A 1 protein and a variant or mutation leading to a substitution at position 284 of the ATP2A 1 protein.

5. The method according to any one of claims 1 to 4, wherein said at least one *ATP2A1* genetic variant or mutation comprises one or more variant or mutation selected from the group consisting of: a variant or mutation leading to a R143W substitution in the ATP2A1 protein, a variant or mutation leading to a R559C substitution in the ATP2A1 protein, a variant or mutation leading to a R164H substitution in the ATP2A1 protein, a variant or mutation leading to a G211V substitution in the ATP2A 1 protein, a variant or mutation leading to a G286V substitution in the ATP2A1 protein and a variant or mutation leading to a G284V substitution in the ATP2A1 protein.

6. The method according to any one of claims 1 to 5, wherein said at least one *ATP2A1* genetic variant or mutation comprises a variant or mutation leading to a substitution at position 143 of the ATP2A1 protein, and/or a variant or mutation leading to a substitution at position 559 of the ATP2A 1 protein.

7. The method according to any one of claims 1 to 6, wherein said at least one *ATP2A1* genetic variant or mutation comprises a variant or mutation leading to a R143W substitution in the ATP2A1 protein, and/or or a variant or mutation leading to a R559C substitution in the ATP2A1 protein.

8. The method according to any one of claims 1 to 7, wherein said *ATP2A1* genetic variant or mutation is a single nucleotide polymorphism (SNP) or a point mutation.

9. The method according to any one of claims 1 to 8, further comprising determining whether the bovine is heterozygous, compound heterozygous, or homozygous for the at least one *ATP2A1* genetic variant or mutation.

10. The method according to any one of claims 1 to 9, wherein the heterozygous or homozygous presence of the at least one *ATP2A1* genetic variant or mutation is indicative of the bovine having or being susceptible to Dry Meat Syndrome.

11. The method according to any one of claims 1 to 10, wherein the nucleic acid is DNA or RNA.

12. The method according to any one of claims 1 to 11, wherein the nucleic acid is from a sample selected from the group consisting of blood, sperm, hair roots, milk, body fluids, tissues including nucleated cells, and combinations thereof.

13. The method according to any one of claims 1 to 12, wherein the bovine is *Bos taurus.*

14. The method according to any one of claims 1 to 13, wherein the bovine is of a breed selected from the group consisting of Belgian Blue, Charolais, Roodbont, Piedmontese, Chianina, and Romagnola.

15. A method for selecting a bovine animal for meat quality using the method according to any one of claims 1 to 14.

## Patentansprüche

1. Verfahren zur Bestimmung, ob ein Rind von TDMS (Tough and Dry Meat Syndrome) betroffen oder dafür anfällig ist, umfassend Bestimmen des Vorliegens oder Fehlens wenigstens einer genetischen Variante oder Mutation im ATP2A1-Gen in einer Nukleinsäure von dem Rind.

2. Verfahren nach Anspruch 1, wobei das Vorliegen oder Fehlen der wenigstens einen genetischen Variante oder Mutation von *ATP2A1* durch Genotypisieren oder Sequenzieren wenigstens eines Teils des ATP2A1-Gens bestimmt wird.

3. Verfahren nach Anspruch 1 oder 2, wobei die wenigstens eine genetische Variante oder Mutation von *ATP2A1* eine hypomorphe Mutation im ATP2A1-Gen umfasst.

4. Verfahren nach einem der Ansprüche 1 bis 3, wobei die wenigstens eine genetische Variante oder Mutation von *ATP2A1* eine oder mehrere Varianten oder Mutationen umfasst, die ausgewählt sind aus der Gruppe bestehend aus: einer Variante oder Mutation, die zu einer Substitution an Position 143 des ATP2A1-Proteins führt, einer Variante oder Mutation, die zu einer Substitution an Position 559 des ATP2A1-Proteins führt, einer Variante oder Mutation, die zu einer Substitution an Position 164 des ATP2A1-Proteins führt, einer Variante oder Mutation, die zu einer Substitution an Position 211 des ATP2A1-Proteins führt, einer Variante oder Mutation, die zu einer Substitution an Position 286 des ATP2A1-Proteins führt und einer Variante oder Mutation, die zu einer Substitution an Position 284 des ATP2A1-Proteins führt.

5. Verfahren nach einem der Ansprüche 1 bis 4, wobei die wenigstens eine genetische Variante oder Mutation von *ATP2A1* eine oder mehrere Varianten oder Mutationen umfasst, die ausgewählt sind aus der Gruppe bestehend aus: einer Variante oder Mutation, die zu einer R143W-Substitution im ATP2A1-Protein führt, einer Variante oder Mutation, die zu einer R559C-Substitution im ATP2A1-Protein führt, einer Variante oder Mutation, die zu einer R164H-Substitution im ATP2A1-Protein führt, einer Variante oder Mutation, die zu einer G211V-Substitution im ATP2A1-Protein führt, einer Variante oder Mutation, die zu einer G286V-Substitution im ATP2A1-Protein führt und einer Variante oder Mutation, die zu einer G284V-Substitution im ATP2A1-Protein führt.

6. Verfahren nach einem der Ansprüche 1 bis 5, wobei die wenigstens eine genetische Variante oder Mutation von *ATP2A1* eine Variante oder Mutation, die zu einer Substitution an Position 143 des ATP2A1-Proteins führt, und/oder eine Variante oder Mutation, die zu einer Substitution an Position 559 des ATP2A1-Proteins führt, umfasst.

7. Verfahren nach einem der Ansprüche 1 bis 6, wobei die wenigstens eine genetische Variante oder Mutation von *ATP2A1* eine Variante oder Mutation, die zu einer R143W-Substitution im ATP2A1-Protein führt, und/oder eine Variante oder Mutation, die zu einer R559C-Substitution im ATP2A1-Protein führt, umfasst.

8. Verfahren nach einem der Ansprüche 1 bis 7, wobei es sich bei der genetischen Variante oder Mutation von *ATP2A1* um einen Einzelnukleotid-Polymorphismus (Single Nucleotide Polymorphism, SNP) oder eine Punktmutation handelt.

9. Verfahren nach einem der Ansprüche 1 bis 8, ferner umfassend Bestimmen, ob das Rind heterozygot, komplex heterozygot oder homozygot für die wenigstens eine genetische Variante oder Mutation von *ATP2A1* ist.

10. Verfahren nach einem der Ansprüche 1 bis 9, wobei das heterozygote oder homozygote Vorliegen der wenigstens einen genetischen Variante oder Mutation von *ATP2A1* anzeigt, dass bei dem Rind Dry Meat Syndrome vorliegt oder eine Anfälligkeit dafür besteht.

11. Verfahren nach einem der Ansprüche 1 bis 10, wobei es sich bei der Nukleinsäure um DNA oder RNA handelt.

12. Verfahren nach einem der Ansprüche 1 bis 11, wobei die Nukleinsäure aus einer Probe stammt, die aus der Gruppe bestehend aus Blut, Sperma, Haarwurzeln, Milch, Körperflüssigkeiten, kernhaltige Zellen enthaltenden Geweben und Kombinationen davon ausgewählt ist.

13. Verfahren nach einem der Ansprüche 1 bis 12, wobei es sich bei dem Rind um *Bos taurus* handelt.

14. Verfahren nach einem der Ansprüche 1 bis 13, wobei das Rind einer Rasse angehört, die aus der Gruppe bestehend aus Belgian Blue, Charolais, Roodbont, Piedmontese, Chianina und Romagnola ausgewählt ist.

15. Verfahren zur Auswahl eines Rindes nach Fleischqualität unter Verwendung des Verfahrens nach einem der Ansprüche 1 bis 14.

## Revendications

1. Procédé permettant de déterminer si un bovin est affecté ou sensible au syndrome de la viande dure et sèche (TDMS), comprenant la détermination de la présence ou de l'absence d'au moins un(e) variant ou mutation génétique dans le gène *ATP2A1* dans un acide nucléique provenant du bovin.

2. Procédé selon la revendication 1, dans lequel la présence ou l'absence de l'au moins un(e) variant ou mutation génétique *ATP2A1* est déterminée par génotypage ou séquençage d'au moins une partie du gène *ATP2A1.*

3. Procédé selon la revendication 1 ou 2, dans lequel ledit/ladite au moins un(e) variant ou mutation génétique *ATP2Al* comprend une mutation hypomorphique dans le gène *ATP2Al.*

4. Procédé selon l'une quelconque des revendications 1 à 3, dans lequel ledit/ladite au moins un(e) variant ou mutation génétique *ATP2A1* comprend un(e) ou plusieurs variants ou mutations sélectionné(e)s dans le groupe constitué par : un variant ou une mutation conduisant à une substitution en position 143 de la protéine ATP2A1, un variant ou une mutation conduisant à une substitution en position 559 de la protéine ATP2A1, un variant ou une mutation conduisant à une substitution en position 164 de la protéine ATP2A1, un variant ou une mutation conduisant à une substitution en position 211 de la protéine ATP2A1, un variant ou une mutation conduisant à une substitution en position 286 de la protéine ATP2A1 et un variant ou une mutation conduisant à une substitution en position 284 de la protéine ATP2A1.

5. Procédé selon l'une quelconque des revendications 1 à 4, dans lequel ledit/ladite au moins un(e) variant ou mutation génétique *ATP2A1* comprend un(e) ou plusieurs variants ou mutations sélectionné(e)s dans le groupe constitué par : un variant ou une mutation conduisant à une substitution R143W dans la protéine ATP2A1, un variant ou une mutation conduisant à une substitution R559C dans la protéine ATP2A1, un variant ou une mutation conduisant à une substitution R164H dans la protéine ATP2A1, un variant ou une mutation conduisant à une substitution G211V dans la protéine ATP2A1, un variant ou une mutation conduisant à une substitution G286V dans la protéine ATP2A1 et un variant ou une mutation conduisant à une substitution G284V dans la protéine ATP2A1.

6. Procédé selon l'une quelconque des revendications 1 à 5, dans lequel ledit/ladite au moins un(e) variant ou mutation génétique *ATP2A1* comprend un variant ou une mutation conduisant à une substitution en position 143 de la protéine ATP2A1, et/ou un variant ou une mutation conduisant à une substitution en position 559 de la protéine ATP2A1.

7. Procédé selon l'une quelconque des revendications 1 à 6, dans lequel ledit/ladite au moins un(e) variant ou mutation génétique *ATP2A1* comprend un variant ou une mutation conduisant à une substitution R143W dans la protéine ATP2A1, et/ou un variant ou une mutation conduisant à une substitution R559C dans la protéine ATP2A1.

8. Procédé selon l'une quelconque des revendications 1 à 7, dans lequel ledit/ladite au moins un(e) variant ou mutation génétique *ATP2A1* est un polymorphisme de nucléotide simple (SNP) ou une mutation ponctuelle.

9. Procédé selon l'une quelconque des revendications 1 à 8, comprenant en outre la détermination si le bovin est hétérozygote, hétérozygote composé ou homozygote pour l'au moins un(e) variant ou mutation génétique *ATP2A1.*

10. Procédé selon l'une quelconque des revendications 1 à 9, dans lequel la présence hétérozygote ou homozygote de l'au moins un(e) variant ou mutation génétique *ATP2A1* indique que le bovin présente ou est sensible au syndrome de la viande sèche.

11. Procédé selon l'une quelconque des revendications 1 à 10, dans lequel l'acide nucléique est un ADN ou un ARN.

12. Procédé selon l'une quelconque des revendications 1 à 11, dans lequel l'acide nucléique provient d'un échantillon sélectionné dans le groupe constitué par le sang, le sperme, les racines capillaires, le lait, les fluides corporels, les tissus y compris les cellules nucléées, et des combinaisons correspondantes.

13. Procédé selon l'une quelconque des revendications 1 à 12, dans lequel le bovin est *Bos taurus.*

14. Procédé selon l'une quelconque des revendications 1 à 13, dans lequel le bovin est d'une race sélectionnée dans le groupe constitué par le Bleu Belge, le Charolais, le Roodbont, le Piémontais, le Chianina et le Romagnola.

15. Procédé pour la sélection d'un animal bovin pour la qualité de la viande en utilisant le procédé selon l'une quelconque des revendications 1 à 14.
